# EUROPÄISCHE PATENTANMELDUNG

(11) **EP 2 918 282 A1**
(43) Veröffentlichungstag der Anmeldung: **16.09.2015**
(21) Anmeldenummer: 14197569.8
(22) Anmeldetag: 12.12.2014
(51) Int. Cl.: A61K 38/05, A61K 31/704, A61P 31/16

(54) **Pharmazeutisches Mittel zur Behandlung von virusbedingten Krankheiten, wie Grippe**

(30) Priorität: 14.03.2014 RU 2014109986
(71) Anmelder: OOO "CytoNIR", 191023 St. Petersburg (RU)
(72) Erfinder: Smirnov, Vjacheslav S., 197374 Sankt-Petersburg (RU)
(74) Vertreter: Jeck, Anton

(57) **Zusammenfassung**

Die Erfindung betrifft ein pharmazeutisches Mittel zur Behandlung von virusbedingten Krankheiten, wie Grippe.

Die Erfindung ist in der Medizin einsetzbar, und zwar für Peptide-Arzneimittel und insbesondere für Präparate, die bei einer Behandlung von Viruskrankheiten, wie Grippe, angewendet werden können.

Das pharmazeutische Mittel enthält einen Induktor eines endogenen Interferons Glutaminsäure-Dipeptid, welches aus einer Gruppe gewählt ist, die α-Glutamyltryptophan, γ-Glutamyltryptophan und α-Lysylglutamyl einschließt, und einen Koinduktor eines endogenen Interferons Glycyrrhizinsäure in Form eines Natriumsalzes oder eines Ammoniumsalzes nach der Erfindung in folgenden Mengen: Dipeptid 0,02 - 0,2 mg/kg des Körpergewichts des Patienten, Glycyrrhizinat 0,2 - 2,0 mg/kg des Körpergewichts des Patienten.

Damit ist mit diesem Mittel die Aufgabe der Erfindung gelöst: Das Mittel kann bei einer Behandlung von beliebigen Arten der Grippe, darunter auch von pandemischer Grippe, unabhängig von ihrem Pathogenitätsgrad erfolgreich angewendet werden.

## Beschreibung

Die Erfindung betrifft ein pharmazeutisches Mittel zur Behandlung von virusbedingten Krankheiten, wie Grippe nach dem Oberbegriff des Anspruchs.

Die Erfindung ist in der Medizin einsetzbar, und zwar für Peptide-Arzneimittel und insbesondere für Präparate, die bei Behandlung von Viruskrankheiten, wie Grippe, und akuten viralen Atemwegserkrankungen eingesetzt werden kann. Diese Krankheiten (insbesondere Grippe) sind an sich ziemlich schwer, rufen oft postinfektiöse Komplikationen, wie z. B. Lungenentzündung, Bronchitis unterschiedlicher Ätiologie, Entzündung von Oberkieferhöhlen oder Stirnhöhlen usw., hervor.

Aus dem Stand der Technik sind Verfahren zur Grippevorbeugung mit Hilfe von exogenen Interferonen des I. Typs bekannt. Es wurde insbesondere gezeigt, dass eine Anwendung von α-Interferon von einer Induktion von virustötenden Zytokinen begleitet wird, welche an einer Verminderung der Grippevirustiter in den Lungen beteiligt sind [Haasbach E, Droebner K, Vogel AB, Planz. O. J Interferon Cytokine Res. 2011 31(6):515-25].

In der RF sind Präparate eines leukozytären humanen Interferons und von rekombinanten Interferonen registriert und werden angewendet. Die Präparate des leukzytären humanen Interferons werden aus Blutserum von gesunden Spendern hergestellt. Deswegen haben diese Präparate häufig eine niedrige Aktivität, welche unzureichend ist, um einen erforderlichen Vorbeugungseffekt, und insbesondere einen therapeutischen Effekt, zu erreichen.

Die rekombinanten Interferon-Präparate werden mittels Gentechnik-Verfahren hergestellt. Sie können einen hohen Aktivitätstiter aufweisen. Ihre Anwendung verursacht aber oft Nebenreaktionen in Form von Fieberzuständen, Arthralgien, Übelkeit, asthenischen Zuständen und etwas seltener in Form von Störungen seitens des zentralen Nervensystems bis zur Entwicklung von Geistesstörungen [F.I. Yershov, O.I. Kiselev. Interferone und ihre Induktoren (von Molekülen bis Arzneimittel), M., 2005; Vcev A. Acta Med Croatica. 2009;63(5):463-7].

Eine Vergleichsanalyse der Wirksamkeit bei verschiedenen Anwendungsbedingungen von Interferonen hat ergeben, dass die höchste Effizienz bei Präventivanwendung (vor der Ansteckung durch einen Virus) der Präparate des exogenen Interferons und nicht bei der Behandlung der eigentlichen Krankheit (nach Anfang der Manifestation) beobachtet wird [Kugel D., Kochs G., Obojes K. et al., J Virol. 2009; 83(8):3843-51].

Die Vorbeugung der Grippe unter Einsatz eines exogenen Interferons ist bei Versuchstieren gut durchführbar, indem das Präparat noch vor der Ansteckung durch einen Virus verabreicht wird. Beim Menschen ist das aber nicht der Fall, denn es ist nicht möglich, den Ansteckungszeitpunkt genau genug festzustellen. Die Einführung von exogenen Interferonen im Hintergrund einer bereits entwickelten Infektion kann sich als unwirksam erweisen, insbesondere zum Zeitpunkt der Herausbildung von Lungenschäden, oder kann sogar eine Verstärkung der Infektion durch kaskadenartige Entwicklung und Aufrechterhaltung eines Zytokinsturms hervorrufen [Chan M.C.W., Cheung CY, Chui W.H. Res. Res. 2005, 6:135-47; Tisoncik J.R., Korth M.J., Simmons, C.P. Microbiol. Mol. Biol. Rev. 2012; 76(1): 16-32].

Ein weiterer Ansatz zur Behandlung von Grippe ist die Anwendung von Präparaten mit einer indirekten Wirkung. Es handelt sich dabei insbesondere um Induktoren (Induktorgene) eines endogenen Interferons [F.I. Yershov, O.I. Kiselev. Interferone und ihre Induktoren (von Molekülen bis zum Arzneimittel), M., 2005]. Das endogene Interferon verkürzt die Krankheitsdauer und reduziert die Menge von postinfektiösen Komplikationen.

Aus dem Stand der Technik sind auch chemische Verbindungen bekannt, die fähig sind, eine Synthese eines endogenen Interferons bei einem Patienten einzuleiten. Es handelt sich dabei um Dipeptide, wie z. B. alpha-Glutamyltryptophan (α-glu-trp), seine ähnlichen Verbindungen gamma-Glutamyltryptophan (γ-glu-trp) und alpha-Lysylgluta-myl (α-lys-glu). Als Induktoren des endogenen Interferons sind Dipeptide wirksamer als Präparate eines exogenen Interferons. Das liegt daran, dass die Einnahme eines Induktors keine überschüssige Bildung eines endogenen Interferons verursacht. Folglich entstehen keine Nebenwirkungen im Zusammenhang mit einer eventuellen Überdosierung. Darüber hinaus bilden sich beim Patienten keine Antikörper, die gegen Fremd-Interferon gerichtet sind [V.G. Morosov und andere. Thymomimetisch wirkende Peptide-Stoffe. St. Petersburg, NAUKA, 2000, S. 19, 33].

Aus dem Stand der Technik ist zudem ein pharmazeutisches Mittel zur Behandlung von virusbedingten Krankheiten bekannt. Dieses Mittel umfasst einen Induktor eines endogenen Interferons Glutaminsäure-Dipeptid (α-glu-trp, γ-glu-trp und α-lys-glu) und einen Koinduktor eines endogenen Interferons Glycyrrhizinsäure in Form eines Natriumsalzes oder eines Ammoniumsalzes. Dabei betragen die Mengen der Zutaten des Mittels (mg pro Dosis) 0,001 - 1,0 für Glutaminsäure-Dipeptid und 5,0 - 500,0 für Glycyrrhizinsäure [RU 2438694, IPC A51K38/05, 2010].

In der Biologie von regelmäßigen Peptiden gibt es eine Auffassung darüber, dass Dipeptide von Glutaminsäure, insbesondere Glutamyltryptophan, in sehr geringen Dosen von max. 0,1 mg bei Parenteralverabreichung wirksam sind [SU 1582393, IPC A61 K, 1989]. Eine ähnliche Ansicht wurde auch hinsichtlich eines anderen Dipeptids γ-D-Glutamyl-L-tryptophan dargelegt [A.A. Kolobov. Struktur- und Funktionsgestaltung, Wirkungsweise und immunbiologische Eigenschaften von γ-D-Glutamyl-L-tryptophan (BESTIM). Diese Zusammenfassung ist eine Dissertation zur Erlangung der Doktorwürde der biologischen Wissenschaften. St. Petersburg, 2008].

Unter Beachtung dieser Angaben wurde während der Entwicklung eines pharmazeutischen Mittels zur Behandlung von virusbedingten Krankheiten nach RU 2438694 vorgeschlagen, Glutaminsäure-Dipeptide in einer Einzeldosis von 0,01 mg zu benutzen. Die Vergrößerung der Dosis des genannten Präparats wurde als unzweckmäßig betrachtet, denn bereits diese Dosis machte es möglich, eine zufriedenstellende Wirkung bei Verabreichung in Kombination mit Glycyrrhizinsäure in den Mengen von 10 - 100 mg im Hintergrund einer Ansteckung von Versuchstieren mit einem Grippevirus A/H2N2/Puerto Rico-8/34 zu erreichen.

Jedoch wurde im Zuge der weiteren Forschungen der Wirksamkeit der patentierten Verschreibung des pharmazeutischen Mittels an einem Grippemodell (verursacht durch einen pandemischen Virenstamm der Grippe A/H1N1pdm09) überraschend festgestellt, dass die vorgeschlagene Verschreibung nicht genug wirksam ist. So betrug die Überlebensrate bei Ansteckung der Versuchstiere mit dem genannten Grippevirusstamm in den Mengen von 1 und 10 DL₅₀ 38% für die Versuchstiere, die mit einem Mittel mit Glutamyltryptophan in der Dosis von 0,01 mg/kg behandelt wurden, und 18 % für Versuchstiere, die mit einem Mittel mit Glycyrrhizinsäure in der Dosis von 10 mg/kg behandelt wurden (bei Vergleichstieren machte die Überlebensrate jeweils 31 % und 9 % aus). Das ist eindeutig unzufriedenstellend.

Der grundsätzliche Unterschied zwischen zwei geprüften Stämmen ist, dass der Stamm A/H2N2/Puerto Rico-8/34 ein Referenz-Grippestamm ist. Infolge zahlreicher Passagen im Körper der Mäuse wurde seine Pathogenität wesentlich abgebaut. Im Grunde genommen kann dieser Stamm nun als eine Abart der üblichen Saison-Grippe eingestuft werden. Der pandemische Virenstamm A/H1N1/Vladivostok/2/09 ist dagegen ein hochpathogener Stamm. Eine Auswertung der klinischen Ausgänge der Grippe bei Menschen hat gezeigt, dass es bestimmte Unterschiede zwischen den Saison- und pandemischen Abarten der Grippe gibt. Die pandemische Grippe lässt sich beachtlich schwerer behandeln und wird oft mit der Entwicklung von tödlichen Komplikationen begleitet, insbesondere in Form von einem akuten Atemnotsyndrom (RDS) [Tran D, Vaudry W, Moore DL, et al. Pediatrics. 2012 Sep; 130(3):397-406; Eriksson CO, Graham DA, Uyeki TM, Randolph AG. Pediatr Crit Care Med. 2012 Nov;13(6):625-31]. Es stellte sich heraus, dass das pharmazeutische Mittel nach RU 2438694 bei virusbedingten Saison-Krankheiten wirksam und bei einer Behandlung der pandemischen Grippe wenig wirksam war.

Es ist Aufgabe der Erfindung, eine wirksame Dosis des pharmazeutischen Mittels zu ermitteln, damit dieses bei einer Behandlung von beliebigen Grippearten, darunter auch bei einer Behandlung von pandemischer Grippe, unabhängig von ihrem Pathogenitätsgrad erfolgreich angewendet werden kann.

Die gestellte Aufgabe wird durch die Merkmale des Anspruchs gelöst.

Das genannte Ziel wird wie folgt erreicht:
Das pharmazeutische Mittel zur Behandlung virusbedingter Krankheiten schließt einen Induktor eines endogenen Interferons Glutaminsäure-Dipeptid, welches aus einer Gruppe gewählt ist, die α-glu-trp, γ-glu-trp und α-lys-glu enthält, und einen Koinduktor eines endogenen Interferons Glycyrrhizinsäure in Form eines Natriumsalzes oder eines Ammoniumsalzes ein, wobei die genannten Verbindungen in folgenden Mengen (mg/kg des Körpergewichts des Patienten) enthalten sind:

| | |
|---|---|
| Dipeptid | 0,02 - 0,2 |
| Glycyrrhizinat (ein Salz der Glycyrrhizinsäure) | 0,2 - 2,0 |

Das Mittel kann auch pharmazeutisch annehmbare Füllstoffe enthalten, darunter Natriumlactat, Saccharose, Fruktose usw.
Das Mittel kann in Form von Kapseln hergestellt werden, die Peptid, Glycyrrhizinat und einen Füllstoff enthalten. Alternativ kann das Mittel (für Kinder) in Form von Sirup oder lösbaren Pulvern hergestellt werden.

Die Wirksamkeit des Mittels wurde an einem Modell der Überlebensfähigkeit von angesteckten Versuchstieren geprüft. Die Versuchstiere wurden mit einer tödlichen Dosis des Grippevirus A/Vladivostok/2/09 (H1N1) angesteckt. Während der Forschung wurde das erfindungsgemäße Mittel 5 Tage lang peroral verabreicht.

Als Versuchstiere wurden weiße männliche Auszucht-Mäuse mit einem Körpergewicht von 16 - 18 g aus der Aufzuchtstation Rappolovo der Russischen Akademie der medizinischen Wissenschaften verwendet. Vor der Prüfung wurden die Versuchstiere einer Quarantäne in einem Vivarium im Laufe einer Woche unterzogen. Die Versuchstiere wurden unter genormten Klima-Bedingungen mit ausgewogener Fütterung gehalten. Jede Gruppe enthielt je 30 Versuchstiere.

Um eine versuchsmäßige Grippe-Infektion zu simulieren, wurde ein bestimmter Virusstamm eingesetzt. Die Versuchstiere wurden mit dem A/Vladivostok/2/09 (H1N1)-Virus in Mengen von 1 und 10 DL₅₀ intranasal angesteckt.

Die Behandlung der angesteckten Mäuse wurde 24 Stunden nach der Ansteckung begonnen. Die Präparate wurden intragastrisch mittels einer Sonde verabreicht. Bei diesen Präparaten handelte es sich um eine Lösung in einer physiologischen Kochsalzlösung. Die Dosis des Arzneimittels wurde pro ein kg des Körpergewichts des Tieres berechnet. Die Behandlung wurde im Laufe von 5 Tagen vorgenommen. Die Wirksamkeit des Mittels wurde anhand der Überlebensraten der Tiere am 21. Tag nach der Ansteckung beurteilt.

Die erste Gruppe der Versuchstiere erhielt eine Lösung von Glutamyltryptophan und Natriumsalz der Glycyrrhizinsäure (nachfolgend GTGS); die zweite Gruppe der Tiere erhielt ein Präparat Tamiflu (Handelsnahme des Präparats Oseltamyvir) in der Dosis von 30 mg/kg; die dritte Gruppe der Tiere erhielt eine physiologische Kochsalzlösung (Placebo).

Die Ergebnisse der Forschung sind der nachstehenden Tabelle zu entnehmen.

Die Forschung hat Folgendes gezeigt:
Das untersuchte Mittel hat Versuchstiere gegen Ansteckung durch einen virulenten Grippevirusstamm wirksam geschützt. So wurden 76,3 % der mit dem genannten Virus in der Dosis von 1 DL₅₀ angesteckten Mäuse infolge der behandlungsmäßigen Verabreichung des Mittels gegen Tod geschützt gegenüber 46,7 % in der Vergleichsgruppe. Da der Virusstamm gegen Oseltamyvir resistent war, wurde keine zuverlässige Schutzsteigerung bei den mit Oseltamyvir behandelten Tieren beobachtet. Die Versuchstiere wurden auch mit dem Virus in der Dosis von 10 DL₅₀ angesteckt. Nach der Gabe des Mittels betrug die Überlebensrate 40 % gegen 93 % Todesfälle bei den nicht behandelten Tieren und gegen 80 % Todesfälle bei Mäusen, die mit Oseltamyvir behandelt wurden.

**Tabelle**

| Mortalitätsentwicklung bei Mäusen während der grippösen Pneumonie, versursacht durch mäuseangepassten pandemischen gegen Oseltamyvir resistenten Grippevirus A/Vladivostok/2/09 (H1N1), unter Bedingungen einer peroralen Einnahme der erforschten Präparate ab 1. bis zum 5. Tag nach der Ansteckung | | | | | | | | |
|---|---|---|---|---|---|---|---|---|
| Präparat | Virus-Dosis | Anzahl der Tiere pro Gruppe | To-des-fälle | Sterblichkeit % | Überleben-de Mäuse | MLD, Tage | Schutzkennzahl, % | MLD-Erhöhung, Tage |
| GTGS | 1 | 15 | 4 | 26,7 | 11 | 13,2 | 50,0 | 2,5 |
| | 10 | 15 | 9 | 60,0 | 6 | 10,6 | 35,7 | 3,5 |
| | Gesamtdosis | 30 | 13 | 43,3 | 17 | 11,9 | 40,9 | 3,0 |
| Tamiflu | 1 | 15 | 6 | 40,0 | 9 | 11,7 | 25,0 | 1,1 |
| | 10 | 15 | 12 | 80,0 | 3 | 9,0 | 14,3 | 1,9 |
| | Gesamtdosis | 30 | 18 | 60,0 | 12 | 10,4 | 18,2 | 1,5 |
| Placebo | 1 | 15 | 8 | 53,3 | 7 | 10,7 | --- | 0,0 |
| | 10 | 15 | 14 | 93,3 | 1 | 7,1 | --- | 0,0 |
| | Gesamtdosis | 30 | 22 | 73,3 | 8 | 8,9 | --- | 0,0 |

| | | | | | | | | |
|---|---|---|---|---|---|---|---|---|
| Hinweis: MLD, Tage - mittlere Lebensdauer innerhalb der Beobachtungsfrist, in Tagen | | | | | | | | |

Die Analyseergebnisse der Pandemie von 2009 haben gezeigt, dass der Virus gegen Oseltamyvir sehr schnell beständig geworden ist. Folglich erwies sich Oseltamyvir als unwirksam (van der Vries E., Schutten M., Fraaij P., et al. Adv Pharmacol. 2013; 67:217-46. Garcia V., Aris-Brosou S. Mol Biol Evol. 2014;31(2):355-63). In diesem Zusammenhang scheint die Annahme begründet zu sein, dass das erfindungsgemäße Mittel unter den Bedingungen einer Herausbildung einer Epidemie einer Grippe, die gegen Oseltamyvir und gegen andere Neuraminidase hemmende Stoffe resistent ist, eine effektive Alternative einer Behandlung mit konventionellen virustötenden Mitteln sein kann.

## Patentansprüche

1. Pharmazeutisches Mittel zur Behandlung von virusbedingten Krankheiten, wie Grippe, mit einem Induktor eines endogenen Interferons Glutaminsäure-Dipeptid, welches aus einer Gruppe gewählt ist, die α-Glutamyltryptophan, y-Glutamyltryp-tophan und α-Lysylglutamyl enthält, und mit einem Koinduktor eines endogenen Interferons Glycyrrhizinsäure in Form eines Natriumsalzes oder eines Ammoniumsalzes,
**dadurch gekennzeichnet,**
**dass** das Mittel die genannten Verbindungen in folgenden Mengen (mg/kg des Körpergewichts des Patienten) enthält:
| | |
|---|---|
| Dipeptid | 0,02 - 0,2 |
| Glycyrrhizinat | 0,2 - 2,0 |
